# EUROPÄISCHE PATENTSCHRIFT

(11) **EP 0 406 169 B1**
(45) Veröffentlichungstag und Bekanntmachung des Hinweises auf die Patenterteilung: **04.06.1997**
(21) Anmeldenummer: 90810463.1
(22) Anmeldetag: 21.06.1990
(51) Int. Cl.: C08K 5/13, C07C 39/06, C09K 15/08

(54) **2,4-Dimethyl-6-s-alkylphenole**
2,4-Dimethyl-6-s-alkyl-phenols
2,4-Diméthyl-6-s-alkyl-phénols

(30) Priorität: 30.06.1989 CH 2435/89
(43) Veröffentlichungstag der Anmeldung: 02.01.1991
(73) Patentinhaber: Ciba Specialty Chemicals Holding Inc., 4057 Basel (CH)
(72) Erfinder: Pitteloud, Rita, Dr., CH-1724 Praroman (CH); Dubs, Paul, Dr., CH-1723 Marly (CH)

(56) Entgegenhaltungen:
- US-A- 3 394 020
- DATABASE CHEMICAL ABSTRACTS (HOST: STN), 1986, Band 105, Nr. 24, Columbus, OH (US); M. BRAVAR et al., AN 210202e
- DATABASE REGISTRY; (HOST: STN), American Chemical Society, Columbus, OH (US); Registry Nr. 69236-48-2
- R. GäCHTER et al., "Taschenbuch der Kunststoff-Additive", 2. Ausgabe, 1983, Carl Hanser Verlag, München-Wien; Seiten 4, 11, 46, 59

## Beschreibung

Die vorliegende Erfindung betrifft Zusammensetzungen, enthaltend ausgewählte organische Polymere, z.B. ein Acrylnitril-Butadien-Styrol-Terpolymer, und 2,4-Dimethyl-6-s-alkylphenole, sowie neue 2,4-Dimethyl-6-s-alkylphenole und damit gegen thermischen, oxidativen und aktinischen Abbau stabilisiertes organisches Material.

Eine Reihe von Trialkylphenolen, zum Beispiel 2,6-Di-tert-butyl-4-methylphenol (®Swanox BHT), und deren Verwendung zum Stabilisieren von organischem Material sind bekannt (vgl. R. Gächter, H. Müller (Hrsg), Taschenbuch der Kunststoff-Additive, 1983, Carl Hanser Verlag, München Wien, S. 4,11,46,59). G. Scott beschreibt in "Atmospheric Oxidation and Antioxidants; Elsevier Publishing Company (1965), Seiten 120-125" den Zusammenhang zwischen stabilisierender Wirkung und Substitution am Phenol für Mineralöle. In US-A-3 511 802 wird die Stabilisierung von Polypropylenharzen mit alkylsubstituierten Phenolen offenbart. Die Herstellung sowie die Spektren von sekundären Alkylphenolen werden in Chemical Abstracts 69:10147s und 72:11860t angegeben.

Aus Chemical Abstracts 105 : 210202e gehen Zusammensetzungen aus Naturkautschuk und Antioxidantien des 2,4,6-Trialkylphenol-Typs wie 2,4-Dimethyl-6-(1-methylpropyl)-phenol hervor.

Die vorliegende Erfindung betrifft Zusammensetzungen enthaltend a) ein Polystyrol, substituiertes Polystyrol oder Co- oder Terpolymeres von Styrol oder substituiertem Styrol, und b) mindestens eine Verbindung der Formel I, 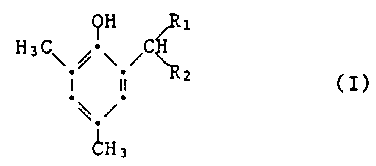 worin R₁ Methyl oder Ethyl bedeutet und R₂ C₂-C₃₀-Alkyl darstellt.

R₂ bedeutet z.B. Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl oder Triacontyl.

Eine bevorzugte Bedeutung von R₂ ist C₈-C₃₀-Alkyl, z.B. C₈-C₁₈-Alkyl oder C₁₀-C₃₀-Alkyl. R₂ als C₁₂-C₁₈-Alkyl ist besonders bevorzugt.

Von Interesse sind Zusammensetzungen, worin die Verbindung der Formel I 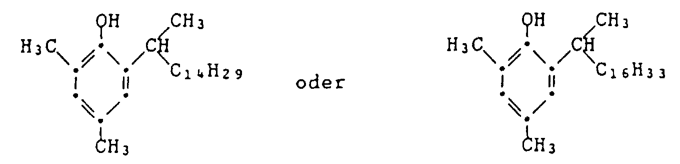 ist.

Ebenfalls von Interesse sind Zusammensetzungen, enthaltend ein Gemisch der Verbindungen 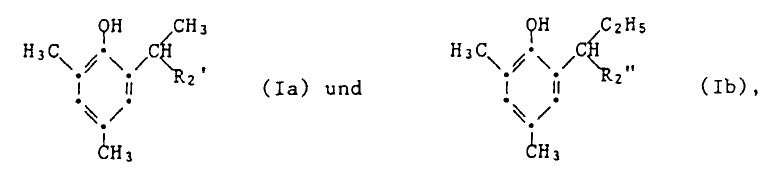 worin R₂' -CₘH₂ₘ₊₁ und R₂" -Cₘ₋₁H₂ₘ₋₁ bedeuten und m eine ganze Zahl von 2-30 darstellt und in den Resten R₂' und R₂" gleich ist.

Das Gewichtsverhältnis der Verbindungen (Ia)/(Ib) ist z.B. 1/99 bis 99/1, bevorzugt 99/1 bis 70/30, insbesondere 95/5 bis 80/20.

Bevorzugt sind auch Zusammensetzungen, worin die Komponente a) ein Polystyrol, substituiertes Polystyrol, Co- oder Terpolymeres von Polystyrol oder substituiertem Polystyrol ist. Als Beispiele seien genannt:
1) Polystyrol, Poly-(p-methylstyrol), Poly-(α-methylstyrol).
2) Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
3) Pfropfcopolymere von Styrol oder α-Methylstyrol, wie z.B. Styrol auf Polybutadien, Styrol auf Polybutadien-Styrol- oder Polybutadien-Acrylnitril-Copolymeren, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 2) genannten Copolymeren, wie sie z.B. als sogenannte ABS-, MBS-, ASA- oder AES-Polymere bekannt sind.

Besonders bevorzugt als Komponente a) sind schlagfestes Polystyrol (IPS), Styrol-Acrylnitril-Copolymere (SAN) und Acryinitril-Butadien-Styrol-Terpolymere (ABS), insbesondere Acrylnitril-Butadien-Styrol-Terpolymere (ABS) und Methylmethacrylat-Butadien-Styrol-Pfropfcopolymere (MBS).

Die Verbindungen der Formel I, worin R₁ Methyl oder Ethyl und R₂ C₁₀-C₃₀-Alkyl bedeuten, sind neu und stellen einen weiteren Gegenstand der Erfindung dar.

Bevorzugte Bedeutungen von R₂ sind den vorangehenden Ausführungen zu entnehmen.

Ebenfalls Gegenstand der Erfindung sind Zusammensetzungen, enthaltend ein gegen oxidativen, thermischen oder aktinischen Abbau empfindliches organisches Material und mindestens eine Verbindung der Formel I, worin R₁ Methyl oder Ethyl ist und R₂ C₁₀-C₃₀-Alkyl bedeutet.

Beispiele für organische Materialien sind die oben unter 1)-3) angegebenen sowie Polycarbonat, Polyestercarbonat, Polyurethan, Polyamid, Copolyamid, Polyacetal und Polyphenylenoxid. Als Beispiele seien genannt:
I) Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
II) Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
III) Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
und
1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE),
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE),
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere,
3a. Kohlenwasserstoffharze (z.B. C₅-C₉) inklusive hydrierte Modifikationen davon (z.B. Klebrigmacherharze),
4. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat,
5. Polymere, die sich von α,β-ungesättigten Säuren und deren Derivaten ableiten, wie Polyacrylate und Polymethacrylate, Polyacrylamide und Polyacrylnitrile,
6. Copolymere der unter 5) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere,
7. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen,
8. Homo- und Copolymere von cyclischen Ethern, wie Polyalkylenglykole, Polyethylenoxyd, Polypropylenoxyd oder deren Copolymere mit Bisglycidylethern,
9. Polyphenylensulfide und deren Mischungen mit Styrolpolymeren oder Polyamiden,
10. Polyharnstoffe, Polyimide, Polyamid-imide und Polybenzimidazole,
11. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester,
12. Polysulfone, Polyethersulfone und Polyetherketone,
13. vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze,
14. trocknende und nicht-trocknende Alkydharze,
15. ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen,
16. vernetzbare Acrylharze, die sich von substituierten Acrylsäureestern ableiten, wie z.B. von Epoxyacrylaten, Urethan-acrylaten oder Polyester-acrylaten,
17. Alkydharze, Polyesterharze und Acrylatharze, die mit Melaminharzen, Harnstoffharzen, Polyisocyanaten oder Epoxidharzen vernetzt sind,
18. vernetzte Epoxidharze, die sich von Polyepoxiden ableiten, z.B. von Bis-glycidylethern oder von cycloaliphatischen Diepoxiden,
19. natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate,
20. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO,
21. natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen und
22. wässrige Emulsionen natürlicher oder synthetischer Kautschuke, wie z.B. Naturkautschuk-Latex oder Latices von carboxylierten Styrol-Butadien-Copolymeren.

Die erfindungsgemässen Zusammensetzungen enthalten zweckmässigerweise 0,01 bis 10 %, bevorzugt 0,05 bis 5 %, insbesondere 0,1 bis 2 % mindestens einer Verbindung der Formel 7, bezogen auf das Gesamtgewicht des zu stabilisierenden organischen Materials.

Neben einer Verbindung der Formel I können die erfindungsgemässen Zusammensetzungen zusätzlich herkömmliche Additive enthalten, wie beispielsweise:
1. Antioxidantien
   1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.
   1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.
   1.3. Hydroxylierte Thiodiphenylether, z.B. 2,2'-Thio-bis-(6-tert.butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).
   1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol), 2,2'-Methylen-bis-[6-(α,α-dimethylbenzyl)-4-nonylphenol], 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat], Bis-(3-tert.butyl-4-hydroxy-5-methylphenyl)-dicyclopentadien, Bis-[2-(3'-tert.butyl-2'-hydroxy-5'-methylbenzyl)-6-tert.butyl-4-methyl-phenyl]-terephthalat.
   1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.
   1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino)-s-triazin, N-(3,5-di-tert.butyl-4-hydroxyphenyl)carbaminsäureoctylester.
   1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.
   1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.
   1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.
   1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.
2. UV-Absorber und Lichtschutzmittel
   2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.
   2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxyzimtsäure-methylester, N-(β-Carbomethoxy-β-cyanovinyl)-2-methyl-indolin.
   2.5. Nickelverbindungen, wie z.B. Nickelkomplexe des 2,2'-Thio-bis-(4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon).
   2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'di-tert.butyloxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o-und p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin.
3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.
4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrittetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.
8. Nukleierungsmittel, wie z.B. 4-tert.Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.
10. Sonstige Zusätze, wie z.B. Weichmacher, Gleitmittel, Emulgatoren, Pigmente, Optische Aufheller, Flammschutzmittel, Antistatika, Treibmittel.

Die Einarbeitung der Verbindungen der Formel I sowie gegebenenfalls weiterer Additive in das organische Material erfolgt nach bekannten Methoden, beispielsweise vor oder während der Formgebung oder auch durch Aufbringen der gelösten oder dispergierten Verbindungen auf das organische Material, gegebenenfalls unter nachträglichem Verdunsten des Lösungsmittels. Die Verbindungen der Formel I können auch in Form eines Masterbatches, der diese beispielsweise in einer Konzentration von 2,5 bis 25 Gew.-% enthält, den zu stabilisierenden Materialien zugesetzt werden.

Die Verbindungen der Formel I können auch vor oder während der Polymerisation oder vor der Vernetzung zugegeben werden.

Die Verbindungen der Formel I können in reiner Form oder in Wachsen, Oelen oder Polymeren verkapselt in das zu stabilisierende Material eingearbeitet werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die Verbindungen der Formel I eignen sich auch als Kettenabbrecher bei der anionischen Lösungspolymerisation von 1,3-Dienen.

Die Verbindungen der Formel I können in Analogie zu bekannten Verfahren hergestellt werden, beispielsweise durch katalytische Alkylierung von 2,4-Xylenol mit α-Olefinen. 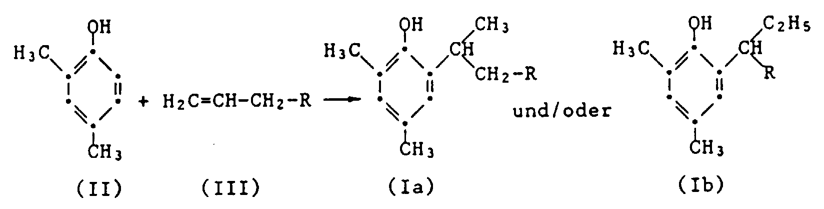 R bedeutet Alkyl. Als Verbindungen der Formel III können auch α-Olefingemische eingesetzt werden, worin R z.B. C₁₃-C₁₇-Alkyl, C₁₇-C₂₁-Alkyl oder C₂₁-C₂₇-Alkyl bedeutet.

Die Umsetzung wird zweckmässigerweise bei Temperaturen von 80-250°C, bevorzugt 130-200°C, in Gegenwart eines Katalysators durchgeführt. Als geeignete Katalysatoren seien genannt:
a) anorganische und organische Säuren, wie z.B. Schwefelsäure oder p-Toluolsulfonsäure;
b) Zeolithe, z.B. ZSM-Zeolith;
c) saure Erden, z.B ®Fulmont 234, ®Fulcat 14 oder ®Fulmont 700;
d) Friedel-Crafts-Katalysatoren, wie z.B in Kozlikovski Ya. B. et al., Zh. Org. Khim. 23, 1918-24 (1987); Laan J.A.M.; Chem. Ind. 1, 34-35 (1987) und Kurashev M.V. et al.; Izv. Akad. Nauk. SSSR, Ser. Khim. 8, 1843-1846 (1986) beschrieben;
e) aktives γ-Aluminiumoxid, wie z.B. in DE-B-1,142,873 und US-A-3,367,981 beschrieben.

Als Katalysator wird aktives γ-Aluminiumoxid besonders bevorzugt.

Wenn bei der Herstellung der Verbindungen der Formel I ein Gemisch aus den Verbindungen (Ia) und (Ib) anfällt, kann dieses z.B. mit Hilfe von chromatographischen Verfahren, insbesondere Gaschromatographie und Hochdruckflüssigchromatographie (HPLC), aufgetrennt werden.

Da die Verbindungen der Formel I bei der Herstellung als Gemische anfallen können, ist ein weiterer Gegenstand der Erfindung ein Gemisch aus den Verbindungen 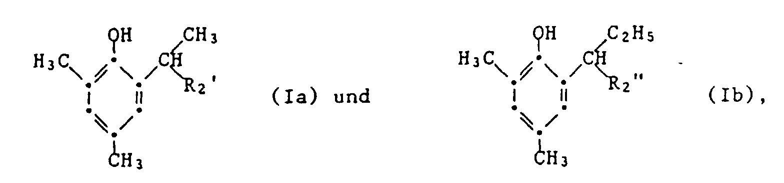 worin R₂' -CₘH₂ₘ₊₁ und R₂" -Cₘ₋₁H₂ₘ₋₁ bedeuten und m eine ganze Zahl von 10-30 ist und in den Resten R₂' und R₂" gleich ist.

Bevorzugt ist ein Gemisch aus den Verbindungen

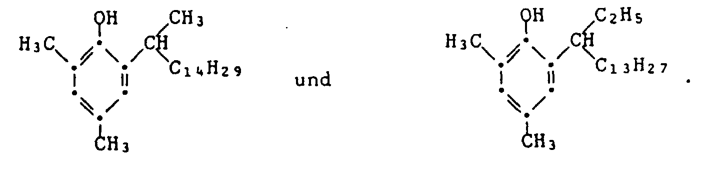

Ebenfalls bevorzugt ist ein Gemisch aus den Verbindungen

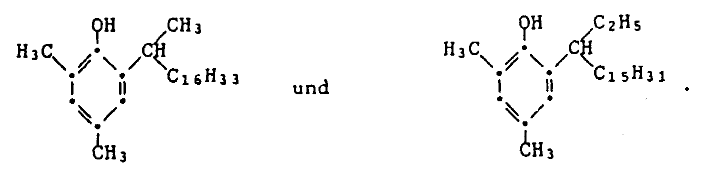

Bevorzugt sind die Verbindungen der Formel I erhältlich durch Umsetzung von 2,4-Xylenol mit einem α-C₁₂-C₃₀-Alken in Gegenwart eines Katalysators.

Die folgenden Beispiele erläutern die Erfindung weiter. Teil- und Prozentangaben beziehen sich darin, soweit nichts anderes angegeben ist, auf das Gewicht.

### Beispiel 1: Herstellung von 2,4-Dimethyl-6-sec-octadecylphenol.

In einem Autoklaven (2000 ml) werden 756 g α-Octadecen (Reinheit: 85 %), 366,5 g 2,4-Xylenol und 30 g aktives γ-Aluminiumoxid (behandelt wie in DE-B-1,142,873, Beispiel 1 beschrieben) als Katalysator eingefüllt. Das Reaktionsgemisch wird auf 310°C erhitzt und 15 Stunden bei dieser Temperatur gerührt. Nach dem Erkalten wird der Katalysator abfiltriert. Das Rohprodukt wird bei 200-225°C und 1 kPa destilliert. Das erhaltene Produkt ist ein farbloses Wachs und liegt als Gemisch von 2,4-Dimethyl-6-(1-methylheptadecyl)phenol und 2,4-Dimethyl-6-(2-ethylhexadecyl)phenol in einem Verhältnis 74/11 vor.

Falls gewünscht, kann das Isomerengemisch mit chromatographischen Methoden (z.B. Gaschromatographie oder Hochdruckflüssigchromatographie) aufgetrennt werden.

Ausbeute: 770 g (=̂ 67 % der Theorie).

Schmelzpunkt:∼ 30°C.

| Elementaranalyse: | | |
|---|---|---|
| Berechnet: | C = 83,35 %; | H = 12,38 %. |
| Gefunden : | C = 83,49 %; | H = 12,32 %. |

### Beispiel 2a: Herstellung von 2,4-Dimethyl-6-sec-hexadecylphenol.

Die Herstellung erfolgt in Analogie zu Beispiel 1. Es werden 672 g α-Hexadecen (Reinheit: 92 %) und 366,5 g 2,4-Xylenol als Reaktanden eingesetzt. Das erhaltene Produkt ist eine farblose Flüssigkeit und liegt als Gemisch von 2,4-Dimethyl-6-(1-methylpentadecyl)phenol und 2,4-Dimethyl-6-(2-ethyltetradecyl)phenol in einem Verhältnis 81/7 vor.

Falls gewünscht, kann das Isomerengemisch mit chromatographischen Methoden aufgetrennt werden.

Ausbeute: 749 g (=̂ 72 % der Theorie).

Siedepunkt: 170-200°C bei 1 kPa.

### Beispiel 2b: Herstellung von 2,4-Dimethyl-6-sec-hexadecylphenol.

In einem Autoklaven von 0,75 l Inhalt werden 220 g (1 Mol) lineares α-Hexadecen (Reinheit: 92 %), 122 g (1 Mol) 2,4-Xylenol und 10 g aktives ZSM-Zeolith als Katalysator vorgelegt. Das Reaktionsgemisch wird auf 220°C geheizt und 15 h bei dieser Temperatur gerührt. Nach dem Erkalten wird der Katalysator abfiltriert und die unreagierten Ausgangsprodukte (Xylenol: Siedepunkt = 40°C bei 10⁻¹ mbar; α-Hexadecen: Siedepunkt = 60-70°C bei 8 x 10⁻² mbar) destillativ entfernt. Anschliessend wird der flüssige Rückstand im Hochvakuum (10⁻² mbar) bei 142-147°C destilliert.

Das Produkt ist eine farblose Flüssigkeit und liegt als Gemisch von 2,4-Dimethyl-6-(1-methylpentadecyl)phenol und 2,4-Dimethyl-6-(2-ethyltetradecyl)phenol in einem Verhältnis von 56/11 vor.

Falls gewünscht, kann das Isomerengemisch mit chromatographischen Methoden aufgetrennt werden.

| Elementaranalyse: | | |
|---|---|---|
| Berechnet: | C = 83,17 %; | H = 12,21 %. |
| Gefunden : | C = 83,14 %; | H = 12,08 %. |

### Beispiel 3a: Herstellung von 2,4-Dimethyl-6-sec-dodecylphenol.

Die Herstellung erfolgt in Analogie zu Beispiel 1. Das erhaltene Produkt liegt als Gemisch von 2,4-Dimethyl-6-(1-methylundecyl)phenol und 2,4-Dimethyl-6-(1-ethyldecyl)phenol in einem Verhältnis von 53/25 vor.

Falls gewünscht, kann das Isomerengemisch mit chromatographischen Methoden aufgetrennt werden.

Siedepunkt: 175°C bei 4 mbar.

### Beispiel 3b: Herstellung von 2,4-Dimethyl-6-sec-dodecylphenol.

Die Herstellung erfolgt in Analogie zu Beispiel 1. Das erhaltene Produkt liegt als Gemisch von 2,4-Dimethyl-6-(1-methylundecyl)phenol und 2,4-Dimethyl-6-(1-ethyldecyl)phenol in einem Verhältnis von 73/15 vor.

Falls gewünscht, kann das Isomerengemisch mit chromatographischen Methoden aufgetrennt werden.

Siedepunkt: 180°C bei 4 mbar.

### Beispiel 4: Herstellung eines Gemisches von 2,4-Dimethyl-6-sec-(C₂₀-C₂₄-alkyl)phenol.

Die Herstellung erfolgt in Analogie zu Beispiel 1. Es werden 2,4-Xylenol und ein α-Olefingemisch (H₂C=CH-CH₂-R mit R=C₁₇-C₂₁-Alkyl) als Reaktanden eingesetzt. Das erhaltene Reaktionsgemisch enthält 2,4-Dimethyl-6-(1-methylnonadecyl)phenol, 2,4-Dimethyl-6-(1-methylhenicosyl)phenol und 2,4-Dimethyl-6-(1-methyltricosyl)phenol im Verhältnis 45/35/3. Das Produkt liegt als viskoses Oel vor.

Falls gewünscht, kann das Gemisch mit chromatographischen Methoden aufgetrennt werden.

| Elementaranalyse: | | |
|---|---|---|
| Berechnet: | C = 83,88 %; | H = 12,84 %. |
| Gefunden : | C = 84,12 %; | H = 13,22 %. |

### Beispiel 5: Herstellung eines Gemisches von 2,4-Dimethyl-6-sec-(C₂₄-C₃₀-alkyl)phenol.

Die Herstellung erfolgt in Analogie zu Beispiel 1. Es werden 2,4-Xylenol und ein α-Olefingemisch (H₂C=CH-CH₂-R mit R=C₂₁-C₂₇-Alkyl) als Reaktanden eingesetzt. Das erhaltene Reaktionsgemisch enthält 2,4-Dimethyl-6-(1-methyltricosyl)phenol, 2,4-Dimethyl-6-(1-methylpentacosyl)phenol, 2,4-Dimethyl-6-(1-methylheptacosyl)phenol und 2,4-Dimethyl-6-(1-methylnonacosyl)phenol im Verhältnis 15/35/33/14. Das Produkt liegt als Wachs vor.

Falls gewünscht, kann das Gemisch mit chromatographischen Methoden aufgetrennt werden.

Schmelzbereiche : 50-60°C.

### Beispiel 6: Stabilisierung von Acrylnitril-Butadien-Styrol-Terpolymer (ABS).

Die in Tabelle 1 bzw. Tabelle 2 angegebenen Additive werden in 40 ml eines Lösungsmittelgemisches aus Hexan/Isopropanol gelöst. Die Lösung wird unter kräftigem Rühren zu einer Dispersion von 100 g ABS in 600 g Wasser gegeben, wobei die Lösung durch das ABS in kurzer Zeit (ca. einer Minute) vollständig absorbiert wird. Das ABS-Pulver wird abgenutscht und während 40 Stunden bei 40°C im Vakuum getrocknet. Dem trockenen Pulver werden 2 % Titandioxid (Pigment), sowie 1 % Ethylen-bis-stearinsäureamid (Gleitmittel) zugegeben. Die Mischung wird anschliessend während 4 Minuten auf einem Zweiwalzenstuhl bei 180°C compoundiert.

Aus dem Walzfell wird bei 175°C eine Platte von 0,8 mm Dicke gepresst, aus welcher Prüflinge von 45x17 mm² ausgestanzt werden. Die Prüfung auf Wirksamkeit der zugesetzten Additive wird durch Hitzealterung in einem Umluftofen bei 180°C vorgenommen. Als Kriterium dient die Farbentwicklung nach 45 Minuten Prüfdauer. Die Farbintensität wird mit dem "Yellowness Index" nach ASTM D 1925-70 bestimmt. Höhere Zahlen bedeuten intensivere Gelbfärbung. Die Versuche zeigen, dass die Gelbfärbung durch die zugesetzten erfindungsgemässen Verbindungen wirksam unterdrückt wird.

**Tabelle 1:**

| Additiv | Yellowness Index nach 45 min bei 180°C |
|---|---|
| - | 78 |
| 0,5 % DLTDP | 75 |
| 0,25 % der Verbindung aus Beispiel 2b + 0,5 % DLTDP | 44 |
| DLTDP: Dilaurylthiodipropionat | |

**Tabelle 2:**

| Additiv | Yellowness Index nach 45 min bei 180°C |
|---|---|
| - | 58 |
| 0,5 % DLTDP | 66 |
| 0,25 % des Gemisches aus Beispiel 4 + 0,5 % DLTDP | 34 |
| 0,25 % des Gemisches aus Beispiel 5 + 0,5 % DLTDP | 36 |
| DLTDP: Dilaurylthiodipropionat | |

### Beispiel 7: Stabilisierung von Methylmethacrylat-Butadien-Styrol-Pfropfcopolymer (MBS).

### Herstellung der Additivemulsion:

Eine Mischung von 6,4 Teilen der Verbindung aus Beispiel 2a, 25,6 Teilen Dilaurylthiodipropionat und 3,4 Teilen Stearinsäure wird solange erhitzt (ca. 80°C) , bis eine Schmelze vorliegt. Unter starkem Rühren wird eine warme Lösung aus 0,4 Teilen festem Natriumhydroxyd und 21,4 Teilen Wasser zugegeben. Zu der erhaltenen Emulsion (Wassertropfen in organischem Medium) werden dann 42,8 Teile warmes Wasser gegeben, wobei die inverse Emulsion erhalten wird. Anschliessend wird die Emulsion mit warmem Wasser auf ¹/₁₀ verdünnt und bis zur Verwendung bei 60°C vorsichtig gerührt.

### Koagulationsbedingungen:

Die in Tabelle 3 angegebene Menge Additivemulsion wird zu 100 ml gekühltem MBS-Latex gegeben. Das erhaltene Gemisch wird 30 Minuten gerührt. Anschliessend wird das Gemisch bei 70°C in 200 ml 0,1 N HCl gegeben, wobei sich eine Temperatur von ca. 60°C einstellt. Unter starkem Rühren werden ca. 20 ml 1 N NaOH hinzugefügt, um einen pH-Wert von 5,5-6 zu erhalten. Dann wird die Suspension auf 95°C erwärmt und 5 Minuten dieser Temperatur ausgesetzt. Schliesslich wird die Suspension gefiltert und das erhaltene feste MBS mit Wasser gewaschen und 48 Stunden bei 60°C im Vakuum getrocknet. Der Durchmesser der MBS-Partikel beträgt 3-10 µm.

Das MBS-Pulver wird an der Luft bei 200°C einer Thermoanalyse unterworfen. Die auftretende exotherme Reaktion ist ein Mass für den Abbau des Polymeren. Das Kriterium für die Stabilisierung ist die Zeit bis zum Eintreten bzw. bis zum Maximum der exothermen Reaktion. Die Ergebnisse sind in der Tabelle 3 angegeben. Der erhaltene Temperaturverlauf zeigt eine gute Stabilisierung des Polymeren.

**Tabelle 3:**

| Konzentration der Additivemulsion in MBS-Latex | Tₒ in Minuten | Tₘ in Minuten |
|---|---|---|
| 1 % *⁾ | 9-11 | 14-16 |
| Tₒ: Zeit bis zum Beginn der exothermen Reaktion | | |
| Tₘ: Zeit bis zum Maximum der exothermen Reaktion | | |

| | | |
|---|---|---|
| *) Dieser Wert entspricht 3 % in trockenem MBS. | | |

## Patentansprüche

1. Zusammensetzung enthaltend a) ein Polystyrol, substituiertes Polystyrol, Co- oder Terpolymeres von Styrol oder substituiertem Styrol, und b) mindestens eine Verbindung der Formel I, 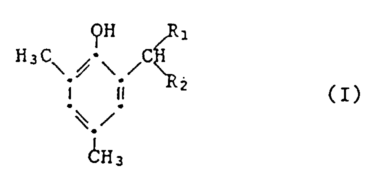 worin R₁ Methyl oder Ethyl bedeutet und R₂ C₂-C₃₀-Alkyl darstellt.

2. Zusammensetzung gemäss Anspruch 1, worin R₂ C₈-C₃₀-Alkyl bedeutet.

3. Zusammensetzung gemäss Anspruch 1, worin R₂ C₁₂-C₁₈-Alkyl ist.

4. Zusammensetzung gemäss Anspruch 1, worin die Verbindung der Formel I 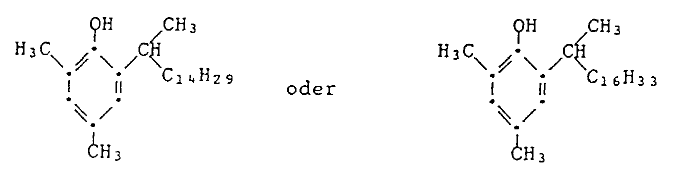 ist.

5. Zusammensetzung gemäss Anspruch 1, enthaltend ein Gemisch der Verbindungen 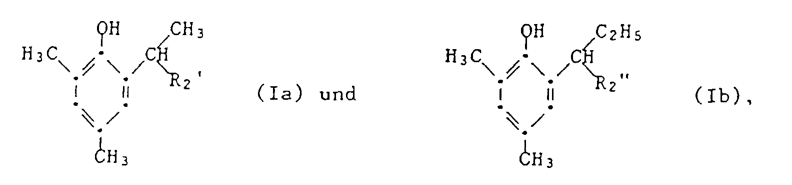 worin R₂' -CₘH₂ₘ₊₁ und R₂" -Cₘ₋₁H₂ₘ₋₁ bedeuten und m eine ganze Zahl von 2-30 darstellt und in den Resten R₂' und R₂" gleich ist.

6. Zusammensetzung gemäss Anspruch 1, worin die Komponente a) schlagfestes Polystyrol (IPS), ein Styrol-Acrylnitril-Copolymer (SAN) oder ein Acrylnitril-Butadien-Styrol-Terpolymer (ABS) ist.

7. Zusammensetzung gemäss Anspruch 1, worin die Komponente a) ein Methylmethacrylat-Butadien-Styrol-Pfropfcopolymer (MBS) ist.

8. Verbindungen der Formel I, 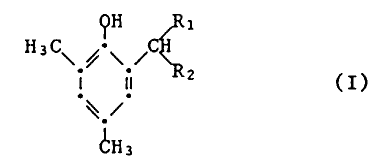 worin R₁ Methyl oder Ethyl und R₂ C₁₀-C₃₀-Alkyl bedeutet.

9. Zusammensetzung enthaltend ein gegen thermischen, oxidativen oder aktinischen Abbau empfindliches organisches Material und mindestens eine Verbindung der Formel I gemäss Anspruch 8.

10. Ein Gemisch der Verbindungen 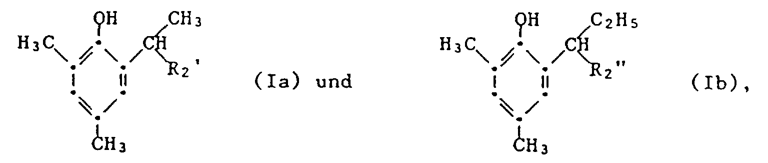 worin R₂' -CₘH₂ₘ₊₁ und R₂" -Cₘ₋₁H₂ₘ₋₁ bedeuten und m eine ganze Zahl von 10-30 ist und in den Resten R₂' und R₂" gleich ist.

11. Ein Gemisch der Verbindungen 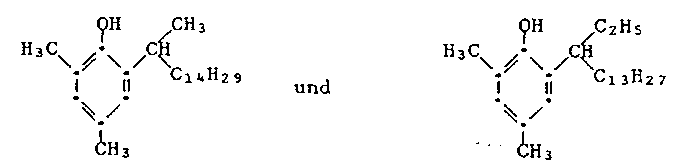 gemäss Anspruch 10.

12. Ein Gemisch der Verbindungen 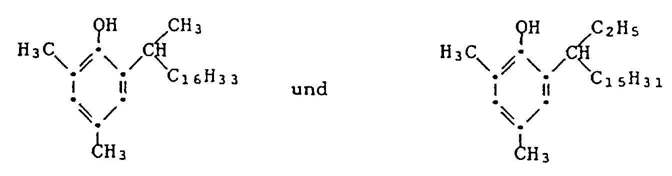 gemäss Anspruch 10.

13. Verfahren zum Stabilisieren von Polystyrol, substituiertem Polystyrol, Co- oder Terpolymeren von Styrol oder substituiertem Styrol, gegen thermischen, oxidativen oder aktinischen Abbau, dadurch gekennzeichnet, dass in diese Materialien mindestens eine Verbindung der in Anspruch 1 definierten Formel I eingearbeitet wird.

## Claims

1. A composition comprising a) a polystyrene, substituted polystyrene, copolymer or terpolymer of styrene or of substituted styrene, and b) at least one compound of the formula I 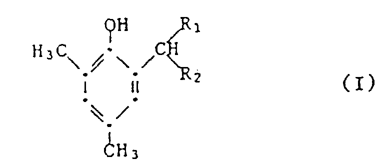 in which R₁ is methyl or ethyl and R₂ is C₂-C₃₀alkyl.

2. A composition according to claim 1, wherein R₂ is C₈-C₃₀alkyl.

3. A composition according to claim 1, wherein R₂ is C₁₂-C₁₈alkyl.

4. A composition according to claim 1, wherein the compound of the formula I is

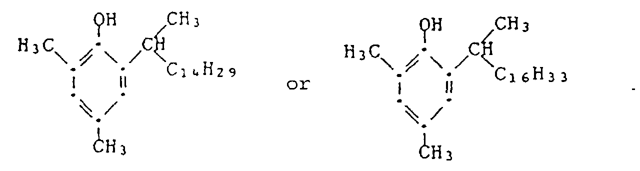

5. A composition according to claim 1, comprising a mixture of the compounds 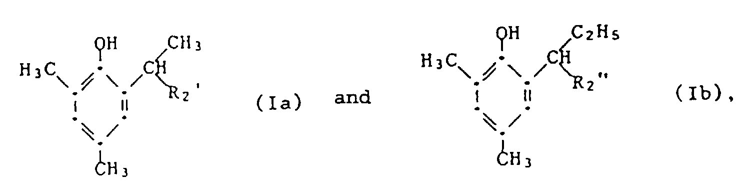 in which R₂' is -CₘH₂ₘ₊₁ and R₂" is -Cₘ₋₁H₂ₘ₋₁ and m is an integer from 2 to 30 and is the same in the radicals R₂' and R₂".

6. A composition according to claim 1, wherein the component a) is impact-resistant polystyrene (IPS), a styrene/acrylonitrile copolymer (SAN) or an acrylonitrile/butadiene/styrene terpolymer (ABS).

7. A composition according to claim 1, wherein the component a) is a methyl methacrylate/butadiene/styrene graft copolymer (MBS).

8. A compound of the formula I 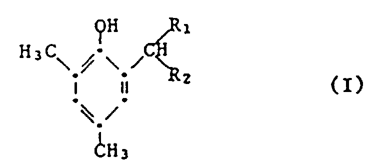 in which R₁ is methyl or ethyl and R₂ is C₁₀-C₃₀alkyl.

9. A composition comprising an organic material which is sensitive to thermal, oxidative or actinic degradation and at least one compound of the formula I according to claim 8.

10. A mixture of the compounds 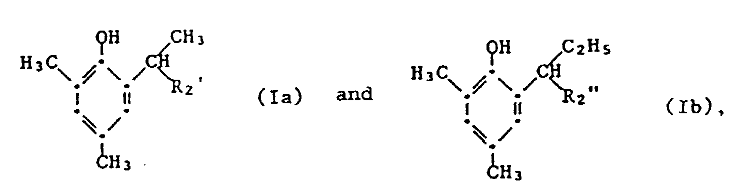 in which R₂' is -CₘH₂ₘ₊₁ and R₂" is -Cₘ₋₁H₂ₘ₋₁ and m is an integer from 10 to 30 and is the same in the radicals R₂' and R₂".

11. A mixture of the compounds 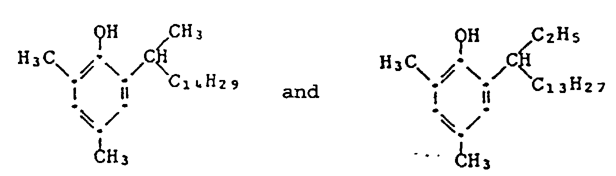 according to claim 10.

12. A mixture of the compounds 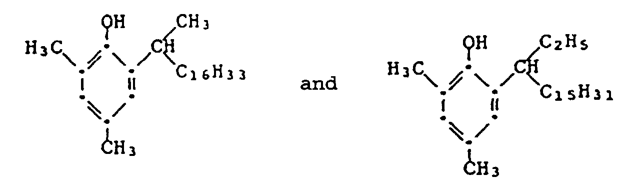 according to claim 10.

13. A process for the stabilization of polystyrene, substituted polystyrene, copolymers or terpolymers of styrene or of substituted styrene against thermal, oxidative or actinic degradation, which comprises incorporating at least one compound of the formula I defined in claim 1 into these materials.

## Revendications

1. Composition contenant
a) un polystyrène, un polystyrène substitué, un copolymère ou terpolymère de styrène ou de styrène substitué, et
b) au moins un composé de formule I 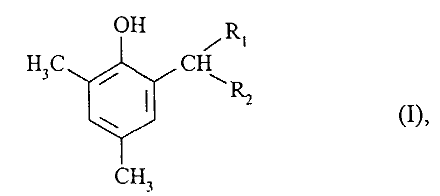 dans laquelle R₁ représente un reste méthyle ou éthyle et R₂ représente un reste alkyle en C₂-C₃₀.

2. Composition selon la revendication 1, dans laquelle R₂ est un reste alkyle en C₈-C₃₀.

3. Composition selon la revendication 1, dans laquelle R₂ est un reste alkyle en C₁₂-C₁₈.

4. Composition selon la revendication 1, dans laquelle le composé de formule I est

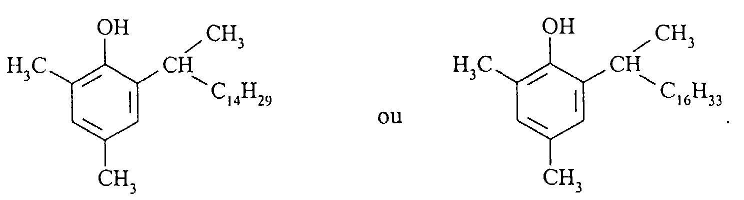

5. Composition selon la revendication 1, contenant un mélange des composés 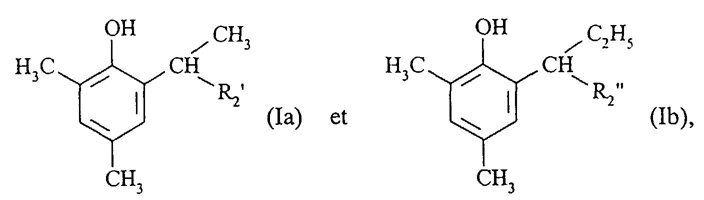 dans lesquelles R₂' est un reste -CₘH₂ₘ₊₁ et R₂" est un reste -Cₘ₋₁H₂ₘ₋₁ et m est un nombre entier de 2 à 30 et est le même dans les restes R₂' et R₂".

6. Composition selon la revendication 1, dans laquelle le constituant a) est un polystyrène résistant au choc (IPS), un copolymère styrène/acrylonitrile (SAN) ou un terpolymère acrylonitrile/butadiène/styrène (ABS).

7. Composition selon la revendication 1, dans laquelle le constituant a) est un copolymère greffé méthacrylate de méthyle/butadiène/styrène (MBS).

8. Composés de formule I 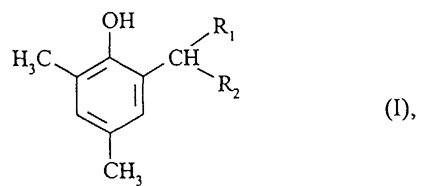 dans laquelle R₁ représente un reste méthyle ou éthyle et R₂ représente un reste alkyle en C₁₀-C₃₀.

9. Composition contenant un matériau organique sensible à la dégradation par la chaleur, l'oxydation ou la lumière, et au moins un composé de formule I selon la revendication 8.

10. Mélange des composés 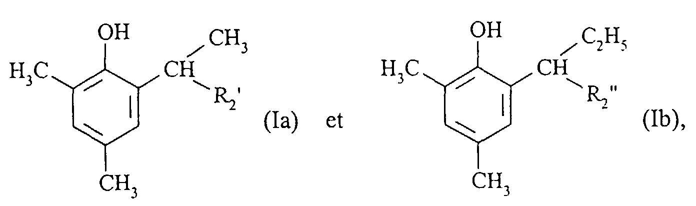 dans lesquelles R₂' est un reste -CₘH₂ₘ₊₁ et R₂" est un reste -Cₘ₋₁H₂ₘ₋₁ et m est un nombre entier de 10 à 30 et est le même dans les restes R₂' et R₂".

11. Mélange des composés 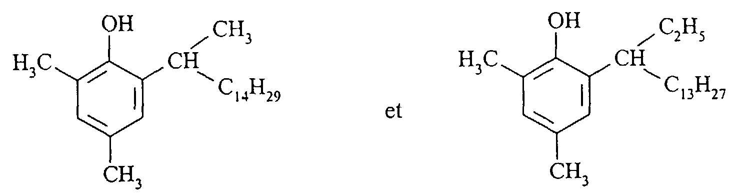 selon la revendication 10.

12. Mélange des composés 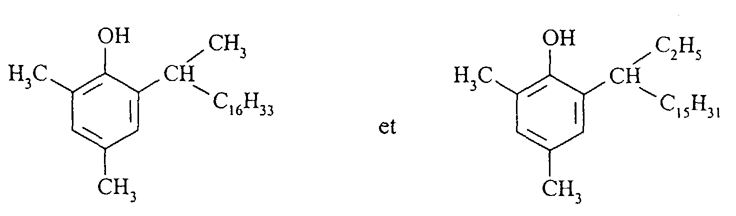 selon la revendication 10.

13. Procédé de stabilisation de polystyrène, de polystyrène substitué, de copolymères ou terpolymères de styrène ou de styrène substitué contre la dégradation par la chaleur, l'oxydation ou la lumière, caractérisé en ce que l'on incorpore dans ces matériaux au moins un composé ayant la formule I définie dans la revendication 1.
